(19) Europäisches Patentamt / European Patent Office / Office européen des brevets

(11) **EP 4 410 191 A1**

(12) # EUROPEAN PATENT APPLICATION
published in accordance with Art. 153(4) EPC

(43) Date of publication:
**07.08.2024 Bulletin 2024/32**

(21) Application number: **22875879.3**

(22) Date of filing: **16.09.2022**

(51) International Patent Classification (IPC):
*A61B 5/107* (2006.01)    *A61B 5/00* (2006.01)

(52) Cooperative Patent Classification (CPC):
**A61B 5/00; A61B 5/107**

(86) International application number:
**PCT/JP2022/034688**

(87) International publication number:
**WO 2023/054008 (06.04.2023 Gazette 2023/14)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **30.09.2021 JP 2021161451**

(71) Applicant: **Shiseido Company, Ltd.**
**Chuo-ku**
**Tokyo 104-0061 (JP)**

(72) Inventor: **EZURE, Tomonobu**
**Tokyo 104-0061 (JP)**

(74) Representative: **Cabinet Beau de Loménie**
**158, rue de l'Université**
**75340 Paris Cedex 07 (FR)**

(54) **METHOD FOR EVALUATING AGING OF SKIN AND APPEARANCE BY HAIR**

(57)    The present disclosure addresses the problem of providing a method for evaluating the state of skin by employing a simple measurement measure, for example a method for evaluating the degree of aging of skin, the elasticity of skin, the sagging of skin or the wrinkling of skin, and the present disclosure provides a skin state evaluation method in which the state of skin is evaluated by employing the state of downy hair on skin as a criterion for the determination. More specifically, the present disclosure relates to a method for evaluating the state of skin by employing, as an evaluation item, at least one of the density of downy hairs, the thickness of downy hair and the length of downy hair. Also provided are an apparatus and program both for performing the evaluation of the state of skin by employing the state of downy hair on skin as a criterion for the determination.

# Fig. 2

100: Device

Image acquisition unit — 110
Image analyzing unit — 120
Skin condition evaluating unit — 130
Results output unit — 140

EP 4 410 191 A1

**Description**

TECHNICAL FIELD

The present invention relates to a method for evaluating the skin condition of a human, and more specifically it relates to a method for evaluating aging of skin and appearance based on hair.

BACKGROUND

Skin aging is the alteration of skin caused by various factors including internal factors such as aging, hormones and metabolism, and external factors such as ultraviolet rays and drying. Skin aging can usually be observed based on exterior appearances such as skin stains, wrinkles, sagging and shrinkage, but aging also proceeds by non-visible changes in the interior skin tissue structure and its constituent components. In PTL 1, for example, aging is evaluated by using blood vitamin D concentration as an index. In PTL 2, expression of genes such as the E-cadherin gene and T-cadherin gene in skin is used as an index. In PTL 3, expression of genes such as OLFML2A and/or CRLF1 in fibroblasts is used as an index.

Although several indicators exist for assessing skin aging, it is important to even more properly evaluate the condition of skin and adopt countermeasures to prevent or improve its aging, and therefore further methods are still desired for evaluating skin condition.

[CITATION LIST]

[PATENT LITERATURE]

[PTL 1] Japanese Unexamined Patent Publication No. 2016-216435
[PTL 2] Japanese Unexamined Patent Publication No. 2010-115131
[PTL 3] Japanese Unexamined Patent Publication No. 2013-116088

SUMMARY

[TECHNICAL PROBLEM]

It is one object of the present disclosure to provide a method for evaluating the condition of skin, such as the extent of skin aging, the elasticity (firmness) of skin, sagging and wrinkles by means of a convenient index for measurement.

[SOLUTION TO PROBLEM]

The present inventors have found that it is possible to evaluate skin condition using an assessment standard based on the condition of downy hair, which is an externally visible feature. The present disclosure therefore provides a method for evaluating skin condition where the assessment standard is the condition of downy hair. More specifically, the present disclosure relates to the following aspects:

[Aspect 1]

A method for evaluating skin condition, wherein the assessment standard is the condition of downy hair.

[Aspect 2]

The method according to aspect 1, which is for evaluation of aging of facial skin.

[Aspect 3]

The method according to aspect 1 or 2, which is for evaluation of skin elasticity.

[Aspect 4]

The method according to any one of aspects 1 to 3, which is for evaluation of skin sagging.

[Aspect 5]

The method according to any one of aspects 1 to 4, which is for evaluation of the condition of wrinkles.

[Aspect 6]

The method according to any one of aspects 1 to 5, wherein the evaluated parameter is at least one from among the density of downy hair, the thickness of downy hair and the length of downy hair.

[Aspect 7]

The method according to any one of aspects 1 to 6, wherein it is evaluated that skin elasticity is high, skin sagging is low or wrinkles are few when the downy hair density is higher than a predetermined reference value, the downy hair thickness is thicker than a predetermined reference value and/or the downy hair length is longer than a predetermined reference value.

[Aspect 8]

The method according to any one of aspects 1 to 7, which is for analysis of a skin image.

[Aspect 9]

A program for executing the method according to any one of aspects 1 to 8 on a computer.

[Aspect 10]

The program according to aspect 9, wherein the method includes a step of analyzing a skin image to obtain at least one measured value from among downy hair density, downy hair thickness and downy hair length.

[Aspect 11]

The program according to aspect 10, which includes a step of comparing the measured value with a predetermined reference value.

[Aspect 12]

A skin condition evaluating device, wherein skin condition is evaluated based on the condition of downy hair.

[Aspect 13]

The device according to aspect 11, which includes:

an image acquisition unit that acquires an image of skin,
an image analyzing unit that analyzes the acquired image of skin,
a skin condition evaluating unit that evaluates the skin condition based on the analysis results and
a results output unit that outputs the evaluation results.

[Aspect 14]

The device according to aspect 13, wherein the image analyzing unit generates at least one measured value from among downy hair density, downy hair thickness and downy hair length.

[Aspect 15]

The device according to aspect 14, wherein the skin condition evaluating unit carries out evaluation by comparing the measured value with the predetermined reference value.

BRIEF DESCRIPTION OF DRAWINGS

Fig. 1 shows a 6-level assessment scale for the condition of downy hair.
Fig. 2 shows an example of the construction of the device of the present disclosure.
Fig. 3 shows an example of the construction of the system of the present disclosure.
Fig. 4 is a diagram showing an example of a hardware configuration that can carry out processing according to the present disclosure.
Fig. 5 is a flow chart showing an example of processing (a program) carried out by the device of the present disclosure.
Fig. 6 is a graph showing correlation between average value for cheek downy hair length and cheek skin grade.

DESCRIPTION OF EMBODIMENTS

[Method for evaluating skin condition]

One aspect of the disclosure relates to a method for evaluating skin condition wherein the assessment standard is the condition of downy hair. As mentioned above, the present inventors found that the condition of skin, including the degree of aging, skin elasticity (skin elasticity also being referred to as "firmness"), skin sagging and wrinkles (wrinkles being formed by loss of skin elasticity) can be evaluated by examining the condition of downy hair on the skin, and especially the condition of downy hair on facial skin. For example, more downy hair is evaluated as higher skin elasticity. More downy hairs is also evaluated as less skin sagging or fewer wrinkles. Conversely, less downy hair is evaluated as progressed skin aging. As used herein, "downy hair" refers to very soft thin hair growing on the human face or nape of the neck, and is distinguished from beard, eyebrows, eyelashes and head hair. Wrinkles are generally described by frequency (few or many, for example) and degree (shallow or deep, for example), but as used herein, "few wrinkles" includes "few and/or shallow wrinkles".
More specifically, according to one aspect of the disclosure, the condition of skin may be evaluated based on at least one evaluated parameter from among downy hair density, downy hair thickness and downy hair length. These parameters can be evaluated by analyzing an image of skin using a computer. A combination of two or three of these parameters may also be evaluated, or a different parameter from these may also be combined for evaluation. The site used for evaluation may be facial skin, and especially skin from the cheek area.
The downy hair density, downy hair thickness and downy hair length may each be absolute values or relative values. A relative value can be calculated as a ratio with respect to pixels obtained in image analysis, for example. The downy hair density, downy hair thickness and downy hair length may also each be average values. An average value can be calculated by evaluating a site several times, measuring the values of downy hair density, downy hair thickness or downy hair length, and averaging the measured values.
Evaluation of skin condition may also be made by stratification and classification of the condition of downy hair. For example, assessment of the condition of downy hair may be made based on the following 6-level stratified scale.

0 - No hair found
1 - Very thin hairs found, sparsely dispersed
2 - Thin and short hairs found, sparsely dispersed
3 - Thin and long hairs found, sparsely dispersed
4 - Thin and long hairs found.
5 - Thick and long hairs found.

The standard for thickness in classifying "very thin hairs", "thin hairs" and "thick hairs" may be determined as appropriate by a person skilled in the art. The standard for density in classifying "no hair found", "hairs found, sparsely dispersed" and "hair found" may likewise be determined as appropriate by a person skilled in the art. The standard for length in classifying "short hairs" and "long hairs" may similarly be determined as appropriate by a person skilled in the art. For example, a person skilled in the art can classify downy hair thickness in several sampled images using 3 levels, based on visual assessment. A person skilled in the art can likewise classify density and length using 3 levels based on visual assessment. The number of levels is not limited to 3 and may be set as desired.

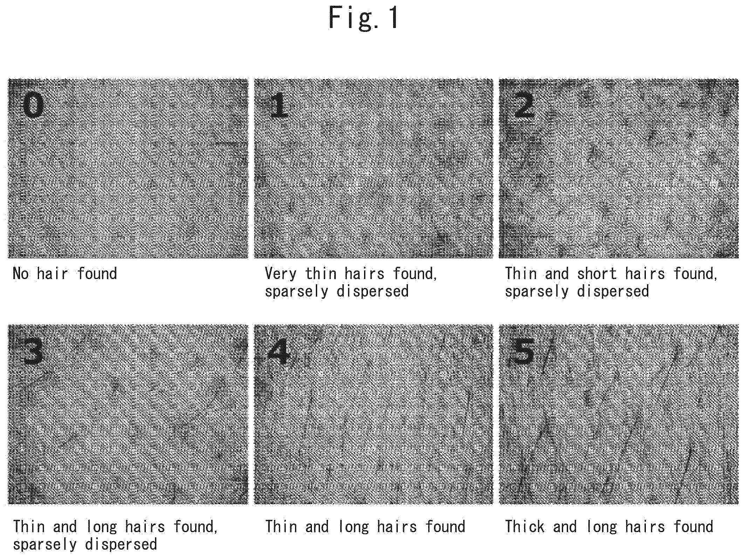

Fig. 1 shows an example of a condition of downy hair in the cheek area, classified on a scale with 6 levels.

According to one aspect of the disclosure, it may be evaluated that skin elasticity is high, skin sagging is low or wrinkles are few and/or shallow, when the downy hair density is higher than a predetermined reference value, the downy hair thickness is thicker than a predetermined reference value and/or the downy hair length is longer than a predetermined reference value. High skin elasticity, low skin sagging and few wrinkles is interpreted as a low degree of skin aging. Naturally, low values for each parameter compared to the reference value may be evaluated as low skin elasticity, high skin sagging or many wrinkles.

For example, high downy hair density compared to the predetermined reference value can be evaluated by acquiring an image of skin and conducting image analysis using a computer, calculating the density by counting the number of downy hairs per fixed area, and comparing it with a reference value. The predetermined reference value may be, for example, the average downy hair density for each age group, or a previous density of the same subject, but there is no limitation to these methods.

Thick downy hair thickness compared to the predetermined reference value can be evaluated, for example, by acquiring an image of skin, conducting image analysis using a computer, calculating the average value of the downy hair thickness in the image, and comparing it with a reference value. The predetermined reference value may be, for example, the average downy hair thickness for each age group, or the previous average downy hair thickness of the same subject, but there is no limitation to these methods.

Long downy hair length compared to the predetermined reference value can be evaluated, for example, by acquiring an image of skin, conducting image analysis using a computer, calculating the average value of the downy hair length in the image, and comparing it with a reference value. The predetermined reference value may be, for example, the average downy hair length for each age group, or the previous average downy hair length of the same subject, but there is no limitation to these methods.

According to one aspect of the disclosure, the condition of downy hair (s) may be evaluated based on an index calculated by weighting and summing the digitized downy hair density (d), downy hair thickness (t) and downy hair length l. For example, the condition of downy hair (s) can be represented as follows, with the weighted parameters as $w_d$, $w_t$, $w_l$ and C as a constant:

$$s = w_d \times d + w_t \times t + w_l \times l + C \text{ (formula 1)}$$

Each parameter and the constant may be set as desired, with adjustment according to the age and gender of the subject, for example. Each parameter and the constant can also be determined by measuring the condition of downy hair (s) and the downy hair density (d), downy hair thickness (t) or downy hair length (l) of multiple subjects and analyzing correlation based on the measured values. The condition of downy hair (s) obtained in this manner may also be stratified on multiple levels in different predetermined ranges (numerical values for 6 levels from 0 to 5, for example), for use in evaluation. Alternatively, the condition of downy hair (s) calculated in this manner may be directly used as an index of aging. For evaluation of skin condition, such as the degree of skin elasticity or sagging, a correlation function between the condition of downy hair (s) and numerical values representing skin condition may be used to calculate a numerical value representing skin condition. The correlation function between condition of downy hair (s) and numerical values representing skin condition can be determined by measuring the numerical values representing the condition of downy hair (s) and skin condition for multiple subjects, for example, and analyzing correlation based on the measured values.

According to one aspect of the disclosure, the method for evaluating skin condition in which the assessment standard is the condition of downy hair includes, for example, (i) a step of acquiring an image of skin of a subject, (ii) a step of analyzing the image and digitizing one or more values for downy hair density, downy hair thickness and downy hair length, and (iii) a step of comparing the one or more values obtained in step (ii) with predetermined reference values. Based on the comparative results of step (iii) it is possible to evaluate skin aging, skin elasticity, skin sagging or condition of wrinkles. The aforementioned steps may be carried out using a computer, and therefore one aspect of the disclosure is a method implemented using a computer.

[Acquisition of skin image]

As a simple description of the method of acquiring a skin image that includes downy hair: first, for example, a facial image of a subject is photographed using a facial image acquiring system comprising a diffused lighting box and digital camera, and a skin image of a specified site (such as the cheek area) is acquired from the acquired facial image. The facial image acquiring system used may be the device described in Registered Utility Model No. 3170125 by the present applicant.

The image from the facial image acquiring system may be taken while irradiating diffused light to reduce the effects of reflection or shadows on the skin surface, and the "color" of the skin at the photographed site may be measured to also acquire color information for the skin. For example, the device described in Japanese Patent No. 6138745 can extract a blemish region from an analysis region in an acquired skin image by image processing of the skin image. A similar image analysis technique may be used to identify downy hair in an image and analyze the downy hair density, downy hair thickness and downy hair length.

[Recommendation method]

One aspect of the disclosure relates to a method in which a subject is recommended an appropriate cosmetic, drug or cosmetic method based on the condition of skin of the subject as evaluated by the method of the disclosure. For example, when an evaluation of reduced skin elasticity (firmness), increased sagging or increased wrinkles is obtained for a subject by the method of the disclosure, the subject may be recommended the use of a cosmetic, drug or cosmetic method suited for improving elasticity, sagging or wrinkles. The cosmetic, drug or cosmetic method recommended based on the condition of skin of the subject may also be recorded in a previously created database.

[Device and system]

One aspect of the disclosure relates to a skin condition evaluating device wherein skin condition is evaluated based on the condition of downy hair. According to one part of this aspect, the device comprises (i) an image acquisition unit that acquires an image of skin, (ii) an image analyzing unit that analyzes the acquired image of skin, (iii) a skin condition evaluating unit that evaluates the skin condition based on the analysis results and (iv) a results output unit that outputs the evaluation results. According to another part of this aspect, each of elements of the device, i.e. the (i) image acquisition unit that acquires an image of skin, (ii) image analyzing unit that analyzes the acquired image of skin, (iii) skin condition evaluating unit that evaluates the skin condition based on the analysis results and (iv) results output unit that outputs the evaluation results, may be present at physically separated locations.

According to another part of this aspect, the device comprises (i) image acquisition means constructed so as to acquire an image of skin, (ii) image analysis means constructed so as to analyze the acquired image of skin, (iii) skin condition evaluating means constructed so as to evaluate skin condition based on the analysis results, and (iv) results output means constructed so as to output the evaluation results.

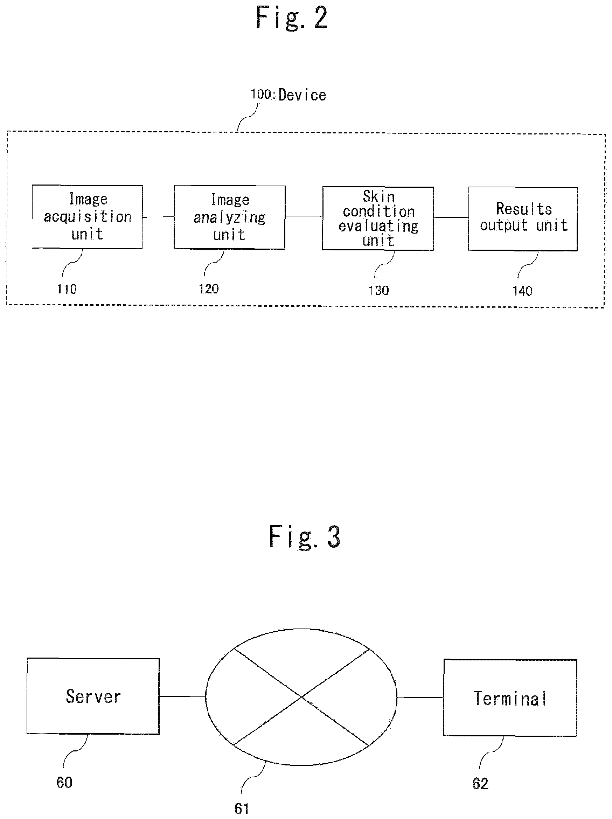

The device 100 shown in Fig. 2 has an image acquisition unit 110, an image analyzing unit 120, a skin condition evaluating unit 130 and a results output unit 140. The device 100 has a computing device such as a CPU (Central Processing Unit), and a storage device such as ROM/RAM, hard disk or flash memory. The storage device is able to store a program for control of the device and data, and acquired data (images, parameters). The computing device can cause the device 100 to function as the image acquisition unit 110, image analyzing unit 120, skin condition evaluating unit 130 and results output unit 140, by executing the program stored in the storage device. That is, the image acquisition unit 110, image analyzing unit 120, skin condition evaluating unit 130 and results output unit 140 may be constructed as functions of the CPU. The device 100 may also operate in cooperation with a computing device and storage device of a server connected by a network (such as a cloud server). The device of the disclosure may considered to be a system constructed with a server 40 and a terminal 42 connected to the server by a network 41 (Fig. 2).

The image acquisition unit 110 can receive skin images from the outside. The image acquisition unit 110 may also include hardware such as a camera which photographs images, illumination means, and a control processor. Alternatively, the image acquisition unit 110 may receive image data from an external device such as a wired or wireless connected camera. For example, the facial image acquiring system described in Registered Utility Model No. 3170125 may be connected to the device of the disclosure in a wireless or wired manner, or it may be integrated as part of the device of the disclosure. The image acquisition unit 110 may also include a database (DB) that stores inputted images.

The image analyzing unit 120 can analyze inputted images and recognize downy hair in a skin image, calculating the number of downy hairs in a fixed area (density), the statistical downy hair thickness and the statistical downy hair length. Any image analysis method or machine learning method known to those skilled in the art may be used for analysis of the images, using a computer.

The skin condition evaluating unit 130 evaluates skin condition based on the data obtained from the image analyzing unit. For example, the skin condition evaluating unit 130 may evaluate that skin elasticity is high, skin sagging is low or wrinkles are few and/or shallow, when the downy hair density is higher than a predetermined reference value, the downy hair thickness is thicker than a predetermined reference value and/or the downy hair length is longer than a predetermined reference value. High skin elasticity, low skin sagging and few wrinkles may be evaluated as a low degree of skin aging. The skin condition evaluating unit 130 may also evaluate that low values for each parameter compared to the reference value indicate low skin elasticity, high skin sagging or many wrinkles.

The skin condition evaluating unit 130 may provide the skin condition evaluation by stratifying and classifying the condition of downy hair on a multi-level scale (such as 6 levels). For example, the condition of downy hair may be assessed by stratifying the condition of downy hair on the following 6-level scale, based on data obtained by the image analyzing unit: 0 - No hair found (for example, the value of the downy hair density is smaller than a predetermined value); 1 - Very thin hairs found, sparsely dispersed (for example, the value of the downy hair density is within a predetermined range and the average downy hair thickness is smaller than a predetermined value); 2 - Thin and short hairs found, sparsely dispersed (for example, the value of the downy hair density is within a predetermined range, the average downy hair thickness is within a predetermined range, and the average downy hair length is less than a predetermined value); 3 - Thin and long hairs found, sparsely dispersed (for example, the value of the downy hair density is within a predetermined range, the average downy hair thickness is within a predetermined range, and the average of the downy hair length is larger than a predetermined value); 4 - Thin and long hairs found (for example, the value of the downy hair density is larger than a predetermined value, the average downy hair thickness is within a predetermined range, and the average downy hair length is larger than a predetermined value); 5 - Thick and long hairs found (for example, the value of the downy hair density is larger than a predetermined value, the average downy hair thickness is larger than a predetermined value, and the average downy hair length is larger than a predetermined value). With stratification in this manner, a smaller value suggests skin aging, lower elasticity, increased sagging or more wrinkles, while a larger value indicates skin with elasticity, less sagging and wrinkles, and younger skin.

The skin condition evaluating unit 130 may calculate the condition of downy hair (s) by weighting and summing the digitized downy hair density (d), downy hair thickness (t) and downy hair length (l). The downy hair density (d), downy hair thickness (t) and downy hair length (l) may each be normalized as well. For example, the condition of downy hair (s) can be represented as follows, with the weighted parameters as $w_d$, $w_t$, $w_l$ and C as a constant:

$$s = w_d \times d + w_t \times t + w_l \times l + C \text{ (formula 1)}$$

Each parameter and the constant may be set as desired, with adjustment according to the age and gender of the subject, for example. Each parameter and the constant can also be determined by measuring the condition of downy hair (s) and the downy hair density (d), downy hair thickness (t) or downy hair length (l) of multiple subjects and analyzing correlation based on the measured values. The condition of downy hair (s) obtained in this manner may also be stratified on multiple levels in different predetermined ranges (numerical values for 6 levels from 0 to 5, for example), for use in evaluation. Alternatively, the condition of downy hair (s) calculated in this manner may be directly used as an index of aging. For evaluation of skin condition, such as the degree of skin elasticity or sagging, a correlation function between the condition of downy hair (s) and numerical values representing skin condition may be used to calculate a numerical value representing skin condition. The correlation function between condition of downy hair (s) and numerical values representing skin condition can be determined by measuring the numerical values representing the condition of downy hair (s) and skin condition for multiple subjects, for example, and analyzing correlation based on the measured values.

According to one embodiment, the skin condition evaluating unit 130 may further include a recommendation unit which recommends a cosmetic, drug or cosmetic method depending on the skin condition evaluation. The recommendation unit may also include a database (DB) that stores information relating to cosmetics, drugs or cosmetic methods. For the purpose of the present specification, the device of the disclosure which includes a recommendation unit may also be referred to as a "recommendation device".

The results output unit 140 may further include a display, printer and speaker for display of information to the user. The results output unit 140 may also output the results to an external device in a wired or wireless manner.

[Hardware configuration]

In the device described above, processing may be carried out by creating an execution program that can execute each function with a computer, and installing the execution program in a general personal computer or server, for example, or a combination thereof.

An example of the hardware configuration of a computer that can carry out processing according to the disclosure will now be explained with reference to the accompanying drawings.

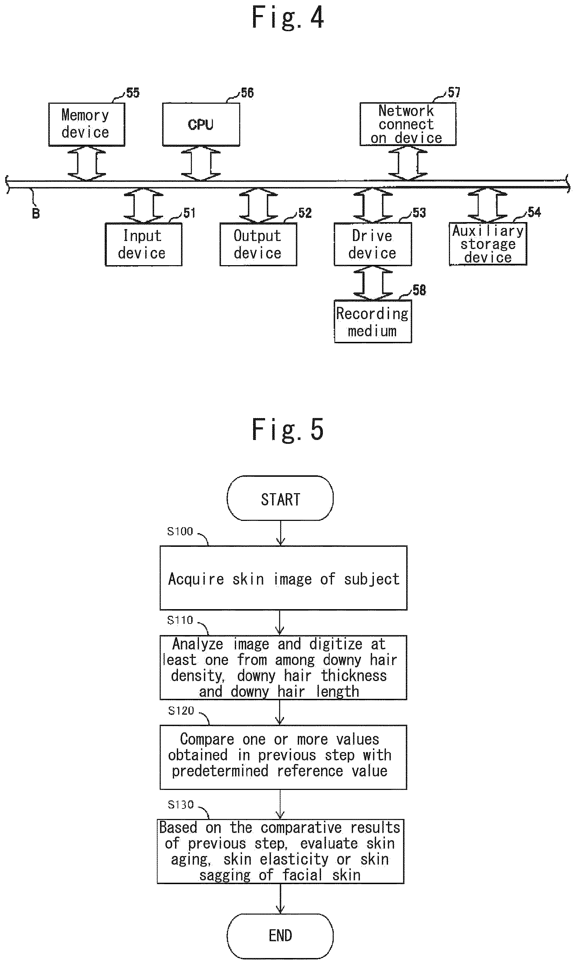

Fig. 4 is a diagram showing an example of a hardware configuration that can carry out processing according to the present disclosure. The computer body shown in Fig. 4 is constructed so as to comprise an input device 51, an output device 52, a drive device 53, an auxiliary storage device 54, a memory device 55, a CPU (Central Processing Unit) 56 which carries out various control operations, and a network connection device 57, all of which are mutually connected with a system bus B.

The input device 51 has a keyboard and a pointing device such as a mouse which are operated by a user, with different operation signals for program execution, for example, being inputted by the user. The input device 51 may also input various data such as facial images of a subject obtained from an external device connected to the network connection device 57, via a communication network.

The output device 52 has a display which displays windows or data necessary for operation of the computer body for processing according to the disclosure, allowing display of the process or results of program execution by the control program in the CPU 56. The output device 52 can also print the processing results on a print medium such as paper for presentation to the user.

The execution program installed in the computer body according to the disclosure is provided, for example, by a recording medium 58, such as a USB (Universal Serial Bus) memory, or a CD-ROM or DVD. The recording medium 58 in which the program is recorded may be set in the drive device 53, the execution program in the recording medium 58 being installed in the auxiliary storage device 54 from the recording medium 58 through the drive device 53.

The auxiliary storage device 54 is storage means such as a hard disk, and it can store the execution program of the disclosure, or a control program provided in a computer, with input/output as necessary.

The memory device 55 stores an execution program read out from the auxiliary storage device 54 by the CPU 56. The memory device 55 consists of a ROM (Read Only Memory) or RAM (Random Access Memory), for example.

All or part of the execution program may also be downloaded from an external device as necessary, via an optional network. An auxiliary storage device 54 in which the execution program is installed and/or a memory device 55 storing the installed execution program, may also be provided in an external device. The external device may be a server. The server may be a specialized server or an undefined server such as a cloud infrastructure. The execution program may be provided by a subscription-type service. A subscription-type service is one form of computer software usage, as a service which is paid for during the period in which the software is used. The manner of usage of the execution program may be in any form, with no particular restrictions.

The CPU 56 can control processing of the entire computer to carry out processing, including computing and input/output of data to the hardware configuration units, based on a control program on an OS (Operating System) and the execution program stored in the memory device 55. The necessary information during execution of the program can be acquired from the auxiliary storage device 54, and the results of execution may be stored.

The network connection device 57 can connect with a communication network or the like to obtain the execution program from another terminal or a server connected to the communication network, or to provide the obtained program execution results or the execution program itself to another terminal or server.

The analysis processing of the disclosure can be executed by such a hardware configuration. Installing the program allows processing according to the disclosure to be easily executed on a general personal computer, smartphone or tablet.

[Program]

One partial aspect of the disclosure relates to a program that causes the method of the disclosure to be executed on a computer. Stated differently, the method of the disclosure may be a method that is fully or partially executed on a computer. According to one part of this aspect, the program of the disclosure may be a program that executes on a computer a process which includes: (i) a step of acquiring an image of skin of a subject (step 100), (ii) a step of analyzing the image and digitizing one or more from among downy hair density, downy hair thickness and downy hair length (step 110), (iii) a step of comparing one or more of the values obtained in step (ii) with predetermined reference values (step 120), and (iv) a step of evaluating skin aging, skin elasticity, skin sagging or condition of wrinkles based on the comparison results of step (iii) (step 130). Fig. 5 shows an example of processing with a device. Steps 100 to 130 are carried out by executing a program by a CPU of the device (optionally including a server).

One partial aspect of the disclosure relates to a computer-readable medium wherein non-transitory commands are stored which, when executed by a processor, carry out the following steps: (i) a step of acquiring an image of skin of a subject (step 100), (ii) a step of analyzing the image and digitizing one or more from among downy hair density, downy hair thickness and downy hair length (step 110), (iii) a step of comparing one or more of the values obtained in step (ii) with predetermined reference values (step 120), and (iv) a step of evaluating skin aging, skin elasticity, skin sagging or condition of wrinkles based on the comparison results of step (iii) (step 130).

According to a partial aspect, in step 110, the program of the disclosure analyzes inputted images and recognizes downy hair in a skin image, calculating the number of downy hairs in a fixed area (density), the statistical downy hair thickness and the statistical downy hair length. Any image analysis method or machine learning method known to those skilled in the art may be used for analysis of the images, using a computer. Identification of downy hair in an image may be by pattern matching or object detection technology using a deep neural network.

According to a partial aspect, in step 110, the program of the disclosure digitizes two or three parameters from among downy hair density, downy hair thickness and downy hair length. From the viewpoint of increasing evaluation precision, preferably the downy hair density, downy hair thickness and downy hair length are all digitized. Other parameters may also be digitized and used for evaluation, in addition to downy hair density, downy hair thickness and downy hair length.

According to a partial aspect, in step 120, the program of the disclosure compares two or three parameters from among downy hair density, downy hair thickness and downy hair length with predetermined reference values. From the viewpoint of increasing evaluation precision, preferably the downy hair density, downy hair thickness and downy hair length are all compared with predetermined reference values.

According to a partial aspect, in step 130 the program of the disclosure may evaluate skin aging, skin elasticity, skin sagging or wrinkles based on the comparison in step 120, for example, stratifying and classifying the condition of downy hair on a multi-level scale (such as a 6-level scale). For example, the condition of downy hair may be assessed by comparing the data obtained in step 110 with the reference values in step 120, and stratifying the condition of downy hair on the following 6-level scale: 0 - No hair found (for example, the value of the downy hair density is smaller than a predetermined value); 1 - Very thin hairs found, sparsely dispersed (for example, the value of the downy hair density is within a predetermined range and the average downy hair thickness is smaller than a predetermined value); 2 - Thin and short hairs found, sparsely dispersed (for example, the value of the downy hair density is within a predetermined range, the average downy hair thickness is within a predetermined range, and the average downy hair length is less than a predetermined value); 3 - Thin and long hairs found, sparsely dispersed (for example, the value of the downy hair density is within a predetermined range, the average downy hair thickness is within a predetermined range, and the average of the downy hair length is larger than a predetermined value); 4 - Thin and long hairs found (for example, the value of the downy hair density is larger than a predetermined value, the average downy hair thickness is within a predetermined range, and the average downy hair length is larger than a predetermined value); 5 - Thick and long hairs found (for example, the value of the downy hair density is larger than a predetermined value, the average downy hair thickness is larger than a predetermined value, and the average downy hair length is larger than a predetermined value). With stratification in this manner, a smaller value is evaluated as greater skin aging, lower elasticity, increased sagging or more wrinkles, while a larger value indicates skin with elasticity, less sagging and wrinkles, and younger skin.

According to a partial aspect, the program of the disclosure may calculate the condition of downy hair (s) by weighting and summing the digitized downy hair density (d), downy hair thickness (t) and downy hair length l, and may use the calculated value for comparison with a reference value. The downy hair density (d), downy hair thickness (t) and downy hair length l may each be normalized as well. For example, the condition of downy hair (s) can be represented as follows, with the weighted parameters as $w_d$, $w_t$, $w_l$ and C as a constant:

$$s = w_d \times d + w_t \times t + w_l \times l + C \text{ (formula 1)}$$

Each parameter and the constant may be set as desired, with adjustment according to the age and gender of the subject, for example. Each parameter and the constant can also be determined by measuring the condition of downy hair (s) and the downy hair density (d), downy hair thickness (t) or downy hair length (l) of multiple subjects and analyzing correlation based on the measured values. The condition of downy hair (s) obtained in this manner may also be stratified on multiple levels in different predetermined ranges (numerical values for 6 levels from 0 to 5, for example), for use in evaluation. Alternatively, the condition of downy hair (s) calculated in this manner may be directly compared with a reference value for the evaluation. For evaluation of skin condition, such as the degree of skin elasticity or sagging, a correlation function between the condition of downy hair (s) and numerical values representing skin condition may be used to calculate a numerical value representing skin condition. The correlation function between condition of downy hair (s) and numerical values representing skin condition can be determined by measuring the numerical values representing the condition of downy hair (s) and skin condition for multiple subjects, for example, and analyzing correlation based on the measured values.

According to a partial aspect, the program of the disclosure may further include in step 130 a step of recommending an appropriate cosmetic, drug or cosmetic method, based on evaluation of the skin condition. In this case, the program of the disclosure refers to a database (DB) storing information for cosmetics, drugs and cosmetic methods. According to a partial aspect, the program of the disclosure may further include, in step 130, outputting the obtained evaluation results or recommendation results. Output of the results may be via any of different devices (such as a display, printer or speaker) connected in a wired or wireless manner.

According to a partial aspect, the program of the disclosure may be installed in a smartphone or tablet for use. In this case, partial processing may also be carried out on a server connected via a network. According to another partial aspect, the program of the disclosure may be installed on the server for use. In this case, the image data may be acquired from a smartphone or tablet connected through the network, and the evaluation results may be outputted to the smartphone or tablet connected through the network.

EXAMPLES

Example 1 Correlation analysis for cheek area

Cheek skin from 30 female subjects of age 30 to 50 was analyzed based on the condition of downy hair and classified on the 6-level scale shown in Fig. 1, assigning points 0 to 5 to each to obtain a grade. When the correlation between condition of downy hair, skin elasticity (Ur/Uf) and degree of sagging was analyzed for each subject, it was found that a higher grade was associated with higher elasticity or a lower degree of sagging, as shown in the following table. Sagging was evaluated by visual assessment of a trained evaluator, while elasticity was measured using a skin viscoelasticity meter (Cutometer) (see Ezure et al., Skin Research and Technology 2009; 15:299-305; the entire description of which is incorporated herein by reference).

[Table 1]

|  | Condition of hair | |
| --- | --- | --- |
|  | Correlation coefficient R | P value |
| Elasticity (Ur/Uf) | 0.4 | <0.05 |
| Sagging | -0.4 | <0.05 |

These data allow evaluation of skin condition including the degree of skin aging, skin elasticity (firmness), wrinkles (which form due to reduced skin elasticity) and sagging, based on the condition of downy hair on the skin.

Example 2 Correlation analysis of downy hair length

Cheek skin from six female subjects was analyzed based on the condition of downy hair and classified on the 6-level scale shown in Fig. 1, assigning points 0 to 5 to each to obtain a grade. Next, skin images were acquired for each of the subjects and image analysis software was used to measure the downy hair length within specified areas, determining the average. The downy hair length was calculated as a relative ratio (ratio with respect to pixels). The average value of cheek downy hair length for each subject measured was analyzed in correlation with the cheek skin grade for each subject.

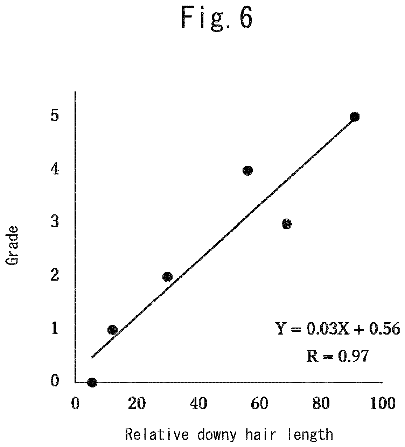

The results are shown in Fig. 6. As shown in Fig. 6, a longer downy hair length was found to be associated with a higher cheek skin grade. The correlation coefficient R was 0.97, as shown in Fig. 6, indicating high correlation. In combination with the results of Example 1, this indicates that downy hair length can be used as a basis for assessment to allow evaluation of skin condition including the degree of skin aging, skin elasticity (firmness), wrinkles (which form due to reduced skin elasticity) and sagging.

All of the publications mentioned throughout the present specification are incorporated herein in their entirety by reference. The technical scope of the disclosure is limited solely by the content of the present Claims, whereas the Examples described herein do not restrict the technical scope in any way. Modifications of the invention, such as additions, deletions or substitutions to the constituent features of the disclosure, are possible so long as the gist of the disclosure is maintained.

REFERENCE SIGNS LIST

    40 Server
    41 Network
    42 Terminal
    51 Input device
    52 Output device
    53 Drive device
    54 Auxiliary storage device
    55 Memory device
    56 CPU
    57 Network connection device
    58 Recording medium
    100 Device
    110 Image acquisition unit
    120 Image analyzing unit
    130 Skin condition evaluating unit
    140 Results output unit

**EP 4 410 191 A1**

**Claims**

1. A method for evaluating skin condition, wherein the assessment standard is the condition of downy hair.

2. The method according to claim 1, which is for evaluation of aging of facial skin.

3. The method according to claim 1 or 2, which is for evaluation of skin elasticity.

4. The method according to any one of claims 1 to 3, which is for evaluation of skin sagging.

5. The method according to any one of claims 1 to 4, which is for evaluation of the condition of wrinkles.

6. The method according to any one of claims 1 to 5, wherein the evaluated parameter is at least one from among the density of downy hair, the thickness of downy hair and the length of downy hair.

7. The method according to any one of claims 1 to 6, wherein it is evaluated that skin elasticity is high, skin sagging is low or wrinkles are few or shallow when the downy hair density is higher than a predetermined reference value, the downy hair thickness is thicker than a predetermined reference value and/or the downy hair length is longer than a predetermined reference value.

8. The method according to any one of claims 1 to 7, which is for analysis of a skin image.

9. A program for executing the method according to any one of claims 1 to 8 on a computer.

10. The program according to claim 9, wherein the method includes a step of analyzing a skin image to obtain at least one measured value from among downy hair density, downy hair thickness and downy hair length.

11. The program according to claim 10, which includes a step of comparing the measured value with a predetermined reference value.

12. A skin condition evaluating device, wherein skin condition is evaluated based on the condition of downy hair.

13. The device according to claim 12, which includes:

    an image acquisition unit that acquires an image of skin,
    an image analyzing unit that analyzes the acquired image of skin,
    a skin condition evaluating unit that evaluates the skin condition based on the analysis results and
    a results output unit that outputs the evaluation results.

14. The device according to claim 13, wherein the image analyzing unit generates at least one measured value from among downy hair density, downy hair thickness and downy hair length.

15. The device according to claim 14, wherein the skin condition evaluating unit carries out evaluation by comparing the measured value with the predetermined reference value.

# Fig.1

0 — No hair found

1 — Very thin hairs found, sparsely dispersed

2 — Thin and short hairs found, sparsely dispersed

3 — Thin and long hairs found, sparsely dispersed

4 — Thin and long hairs found

5 — Thick and long hairs found

EP 4 410 191 A1

# Fig. 2

100:Device

| Image acquisition unit | Image analyzing unit | Skin condition evaluating unit | Results output unit |
|---|---|---|---|
| 110 | 120 | 130 | 140 |

# Fig. 3

| Server | | Terminal |
|---|---|---|
| 60 | 61 | 62 |

# Fig. 4

```
  55              56                        57
┌─────────┐   ┌─────────┐           ┌──────────┐
│ Memory  │   │         │           │ Network  │
│ device  │   │   CPU   │           │ connect  │
│         │   │         │           │ on device│
└────┬────┘   └────┬────┘           └────┬─────┘
     ↕             ↕                      ↕
═════╪═════════════╪══════════════════════╪════════════
  B      ↕             ↕          ↕              ↕
     ┌────┴────┐  ┌────┴────┐┌────┴────┐  ┌──────┴─────┐
     │ Input   │51│ Output  │52│ Drive  │53│ Auxiliary │54
     │ device  │  │ device  │ │ device │  │  storage  │
     │         │  │         │ │        │  │  device   │
     └─────────┘  └─────────┘ └────┬───┘  └───────────┘
                                   ↕  58
                              ┌────┴─────┐
                              │Recording │
                              │ medium   │
                              └──────────┘
```

# Fig. 5

```
                    ┌───────────┐
                    │   START   │
                    └─────┬─────┘
         S100             ↓
      ┌─────────────────────────────────────┐
      │                                      │
      │  Acquire skin image of subject       │
      │                                      │
      └─────────────────────┬────────────────┘
         S110               ↓
      ┌─────────────────────────────────────┐
      │   Analyze image and digitize at      │
      │ least one from among downy hair      │
      │  density,  downy hair  thickness     │
      │     and downy hair length            │
      └─────────────────────┬────────────────┘
         S120               ↓
      ┌─────────────────────────────────────┐
      │  Compare one or more values          │
      │ obtained in previous step with       │
      │ predetermined reference value        │
      └─────────────────────┬────────────────┘
         S130               ↓
      ┌─────────────────────────────────────┐
      │Based on the comparative results      │
      │ of previous step,  evaluate skin     │
      │  aging,  skin elasticity or skin     │
      │     sagging of facial skin           │
      └─────────────────────┬────────────────┘
                            ↓
                    ┌───────────┐
                    │    END    │
                    └───────────┘
```

Fig. 6

$$Y = 0.03X + 0.56$$
$$R = 0.97$$

## INTERNATIONAL SEARCH REPORT

| International application No. |
|---|
| **PCT/JP2022/034688** |

**A. CLASSIFICATION OF SUBJECT MATTER**

***A61B 5/107***(2006.01)i; ***A61B 5/00***(2006.01)i
FI:   A61B5/107 800; A61B5/00 M; A61B5/107 700

According to International Patent Classification (IPC) or to both national classification and IPC

**B. FIELDS SEARCHED**

Minimum documentation searched (classification system followed by classification symbols)

A61B5/107; A61B5/00

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | JP 2006-520614 A (THE GENERAL HOSPITAL CORPORATION) 14 September 2006 (2006-09-14) paragraph [0022] | 1, 6, 9, 12 |
| Y | paragraph [0022] | 8, 10-11, 13-15 |
| A | paragraph [0022] | 3-5, 7 |
| X | "滋賀テックプラングランプリ特別賞を受賞", 学校法人関西文理総合学園長浜バイオ大学 [オンライン], 06 August 2021, [retrieval date 04 October 2022], Internet: <URL:https://www.nagahama-i-bio.ac.jp/research/%e6%bb%8b%e8%b3%80%e3%83%86%e3%83%83%e3%82%af%e3%83%97%e3%83%a9%e3%83%b3%e3%82%b0%e3%83%a9%e3%83%b3%e3%83%97%e3%83%aa%e7%89%b9%e5%88%a5%e8%b3%9e%e3%82%92%e5%8f%97%e8%b3%9e/>, 2nd paragraph 2nd paragraph | 1-2, 9, 12 |
| Y | 2nd paragraph | 8 |
| A | 2nd paragraph, (NAGAHAMA INSTITUTE OF BIO-SCIENCE AND TECHNOLOGY [online]), non-official translation (Received Special Award of Shiga Tech Plan Grand Prix) | 3-7, 10-11, 13-15 |

☑ Further documents are listed in the continuation of Box C.       ☑ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **04 October 2022** | **18 October 2022** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

**INTERNATIONAL SEARCH REPORT**

International application No.

**PCT/JP2022/034688**

| C. | DOCUMENTS CONSIDERED TO BE RELEVANT | |
|---|---|---|
| Category* | Citation of document, with indication, where appropriate, of the relevant passages | Relevant to claim No. |
| Y | JP 2018-200640 A (CANON KK) 20 December 2018 (2018-12-20)<br>paragraphs [0028], [0037], [0063] | 8, 10-11, 13-15 |

Form PCT/ISA/210 (second sheet) (January 2015)

INTERNATIONAL SEARCH REPORT
Information on patent family members

International application No.

**PCT/JP2022/034688**

| Patent document cited in search report | | | Publication date (day/month/year) | Patent family member(s) | | | Publication date (day/month/year) |
|---|---|---|---|---|---|---|---|
| JP | 2006-520614 | A | 14 September 2006 | US 2006/0155266 A1 paragraph [0035] WO 2004/086947 A2 EP 2277470 A1 | | | |
| JP | 2018-200640 | A | 20 December 2018 | (Family: none) | | | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2016216435 A
- JP 2010115131 A
- JP 2013116088 A
- JP 6138745 B

**Non-patent literature cited in the description**

- **EZURE et al.** *Skin Research and Technology,* 2009, vol. 15, 299-305